# EUROPEAN PATENT SPECIFICATION

(11) **EP 3 582 747 B1**
(45) Date of publication and mention of the grant of the patent: **13.10.2021**
(21) Application number: 18700495.7
(22) Date of filing: 16.01.2018
(51) Int. Cl.: A61K 8/35, A61Q 15/00, A61P 29/00

(54) **A PERSONAL CARE COMPOSITION**
KÖRPERPFLEGEZUSAMMENSETZUNG
COMPOSITION DE SOINS PERSONNELS

(30) Priority: 15.02.2017 WO PCT/CN2017/073648; 27.03.2017 EP 17162972
(43) Date of publication of application: 25.12.2019
(73) Proprietor: Unilever Global IP Limited, Wirral, CH62 AZD (GB); Unilever IP Holdings B.V., 3013 AL Rotterdam (NL)
(72) Inventor: EVANS, Richard, Livesey, Bebington Wirral Merseyside CH63 3JW (GB); HARDING, Clive, Roderick, Little Neston Cheshire CH64 4AF (GB); HARICHIAN, Bijan, Trumbull, Connecticut 06611 (US); ROSA, Jose, Guillermo, Trumbull, Connecticut 06611 (US); ZHOU, Luxian, Shanghai 201114 (CN)
(74) Representative: Whaley, Christopher
(86) International application number: PCT/EP2018/050911
(87) International publication number: WO 2018/149578

(56) References cited:
- WO-A1-2010/121007
- FR-A1- 2 838 644
- US-A1- 2010 105 644
- DATABASE WPI Week 200479 Thomson Scientific, London, GB; AN 2004-797340 XP002770481, & CN 1 523 096 A (MASSON GROUP CO LTD) 25 August 2004 (2004-08-25)

## Description

### Field of the invention

The present invention relates to a personal care composition. The invention more particularly relates to a composition and a method of providing antiperspirant and anti-inflammation using certain curcuminoid derivatives. The present invention is especially useful since these curcuminoid derivatives do not have the strong characteristic yellow colour inherent in many curcumin based compounds.

### Background of the invention

The present invention relates to methods and compositions for providing antiperspirant. Antiperspirant actives are added to compositions to reduce perspiration on application to the surface of the body, particularly to the underarm regions of the human body viz. the axilia. Antiperspirant actives are typically astringent metal salts such as those of aluminium or zirconium salts. Antiperspirant actives are usually incorporated in compositions at low pH, in the range of 2 to 7. The present invention relates to developing actives that are not inorganic in nature but are derived from naturally occurring materials.

The present invention concerns identification of actives which could be derivatized from compounds found in extracts of natural materials. Natural materials from which many actives have been extracted include ginger, turmeric, tea, grape, tomato and a host of others. One such active is curcumin which has been known for a long time to alleviate very many health and cosmetic problems. The present inventors have taken curcumin and tried to derivatise it to new compounds hoping to find actives that have anti-perspirant properties. Curcumin is an active that is extractable from the natural rhizome turmeric. Turmeric has been used as a spice in cooking and has a distinctive yellow colour. It is known to have antimicrobial and anti-inflammatory properties. Curcumin has the structure as give below:

| | |
|---|---|
| 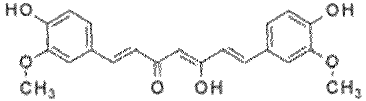 | Enol form |
| 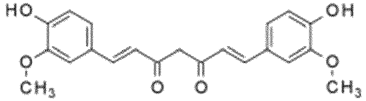 | Keto form |

Although curcumin has very many therapeutic properties which enable it to be used in medical and cosmetic treatments, one of the negatives is that it has a strong yellow colour which impedes the flexibility in preparing cosmetic compositions where visual appeal is very important. The present inventors have tried to retain the structural and spatial attributes of the curcumin backbone while attempting to minimize the yellowness of the synthesized compound.

FR2838644 (L'Oreal, 2003) relates to use of derivatives of 1,7-bisphenyl heptane-3,5-dione (tetrahydrocurcuminoids) as active ingredients in deodorant or antiperspirant cosmetic compositions.

WO2010121007 A1 (Univ Ohio) discloses certain curcumin analogs as Dual JAK2/STAT3 Inhibitors, relevant for detection and treatment of cancer.

US2010105644 A1 (Univ Michigan) discloses a skin augmentation composition comprising a combination of a gingerol and a curcumin.

The compounds synthesized by way of the present invention for inclusion in anti-perspirant compositions is found to have anti-inflammatory benefits.

It is thus an object of the present invention to provide for antiperspirant benefits which could be delivered through cosmetic compositions using actives which are derivatised from naturally occurring materials.

### Summary of the invention

According to the first aspect of the present invention there is provided a personal care composition comprising:
(i) a compound of the Formula 1, Ar-CHₙCHₙ-X.C(R)₂-X.CHₙCHₙ-Ar (Formula 1) wherein Ar is a substituted or unsubstituted phenyl group;
   R is H or CH₃; X is CH(OH) group or C=O group; n has the value 1 or 2; and,
(ii) a topically acceptable base comprising at least 0.1 % of a fragrance wherein, when n=1, the compound of (Formula 1) is 1E,6E)-1,7-bis(3,4 dimethoxyphenyl)-4,4-dimethylhepta-1,6-diene-3,5-dione (Formula 2), 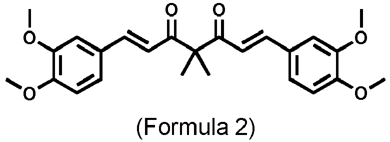 and when n=2, the compound of (Formula 1) is 1,7-bis(4-hydroxy-3 methoxyphenyl) heptane-3,5-diol (Formula 4) 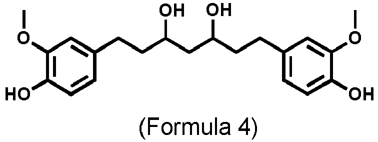 or is 1,7-bis (3,4-dimethoxyphenyl)-4,4-dimethylheptane-3,5-diol (Formula 5).

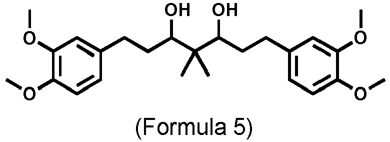

According to another aspect there is provided a personal care composition of the first aspect for use in providing antiperspirant and/or anti-inflammation to a topical surface of a body comprising the step of applying.

According to another aspect there is disclosed the cosmetic, non-therapeutic use of a compound of formulae (2), (4) or (5) as an antiperspirant active.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The compositions of the invention are typically "personal care compositions", suitable for cosmetic use as detailed below. Further, use of the compositions of the invention is typically cosmetic, non-therapeutic use.

In some embodiments of the present invention, the compositions may be used for the therapeutic treatment of hyperhidrosis (extreme sweating).

By "A personal care composition" as used herein, is meant to include a composition for topical application to the skin of mammals, especially humans. Such a composition is preferably of the leave-on type. By a leave-on composition is meant a composition that is applied to the desired skin surface and left on for some time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. It is especially useful for delivering low pH compositions to the axilla of an individual for anti-perspirant benefits. "Skin" as used herein is meant to include skin on any part of the body (e.g., neck, chest, back, arms, underarms, hands, legs, buttocks and scalp) especially the underarms.

The invention relates to a personal care composition comprising:
(i) a compound of the Formula 1,

   Ar-CHₙCHₙ-X.C(R)₂-X.CHₙCHₙ-Ar (Formula 1)

   wherein Ar is a substituted or unsubstituted phenyl group;
   R is H or CH₃;
   X is CH(OH) group or C=O group;
   n has the value 1 or 2; and,
(ii) a topically acceptable base comprising at least 0.1% of a fragrance

When n=1, the compound of (Formula 1) is 1E,6E)-1,7-bis(3,4-dimethoxyphenyl)-4,4-dimethylhepta-1,6-diene-3,5-dione (Formula 2).

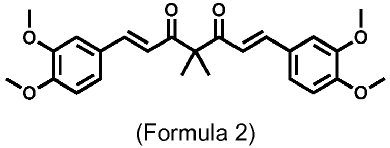

When n=2, the compound of (Formula 1) is 1,7-bis(4-hydroxy-3-methoxyphenyl) heptane-3,5-diol (Formula 4)

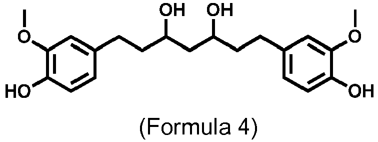

Alternatively, when n=2, the compound of the Formula 1 is 1,7-bis (3,4-dimethoxyphenyl)-4,4-dimethylheptane-3,5-diol (Formula 5).

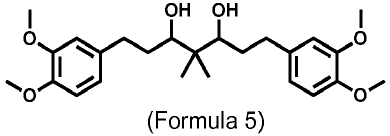

According to another aspect of the present invention there is provided a process (Scheme I) to prepare the compound of formula 2 comprising the following steps:
(i) (step 1): condensation of 3,4-dimethoxybenzaldehyde with 2,4-pentanedione to generate (1E,6E)-1,7-bis(3,4-dimethoxyphenyl)hepta-1,6-diene-3,5-dione; and,
(ii) (step 2): methylation of (1E,6E)-1,7-bis(3,4-dimethoxyphenyl)hepta-1,6-diene-3,5-dione to generate compound with Formula 2.

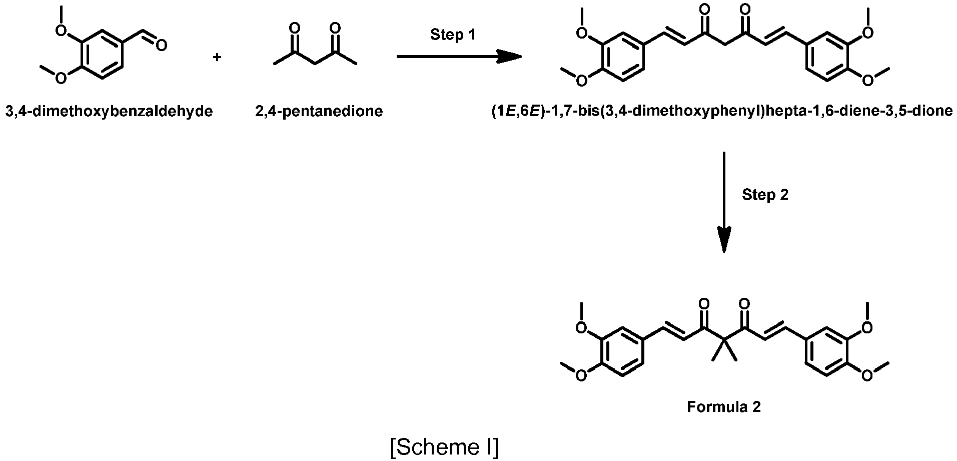

Preferably the compound of Formula 4 is prepared by a process comprising the step of reducing tetrahydrocurcumin using (i) a reducing agent or (ii) via hydrogenation.

### [Scheme 2]

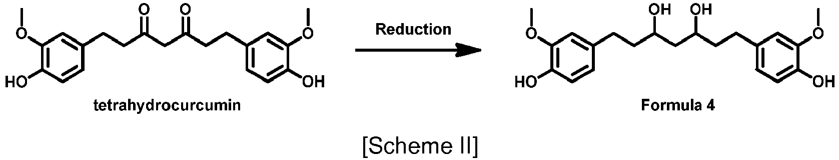

Preferably the compound of Formula 5 is prepared by a process comprising the steps of:
(i) (step 1): methylation of tetrahydrocurcumin with a methylating agent to generate 1 ,7-bis(3,4-dimethoxyphenyl)-4,4-dimethylheptane-3,5-dione; and,
(ii) (step 2): reduction of 1,7-bis(3,4-dimethoxyphenyl)-4,4-dimethylheptane-3,5-dione using a reducing agent to generate compound with Formula 5.

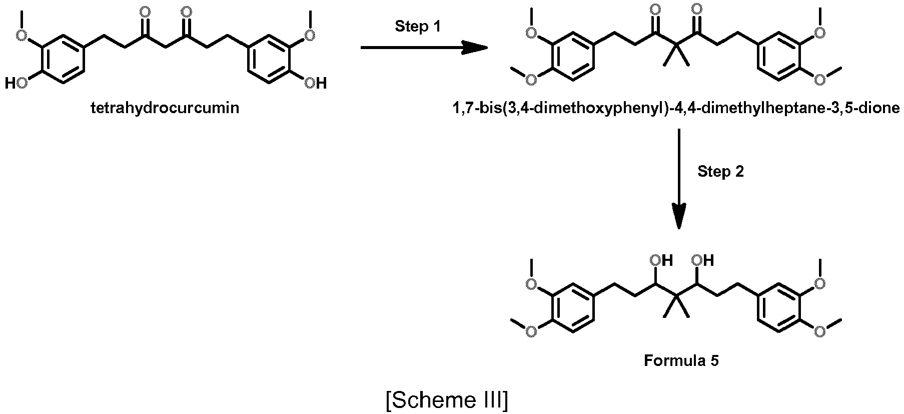

Another aspect of the present invention provides for a personal care composition comprising the compound of formula 1 and a topically acceptable base comprising at least 0.1 % of a fragrance. The various compounds of formula 1, preferred ones being those of formula 2, 4 or 5 are generally included at 0.01 to 5%, preferably at 0.1 to 3%, more preferably at 0.1 to 2% by weight of the composition. The composition of the invention is preferably in the form of a roll-on, a propellant-containing composition, a gel or a stick.

Compositions of the present invention may advantageously comprise an additional antiperspirant active. Whilst this might be a conventional antiperspirant salt comprising Al and/or Zr, such as aluminium chlorohydrate or aluminium-zirconium chlorohydrate optionally complexed with glycine, it is preferred that any additional antiperspirant active is not of this type.

The cosmetic composition of the invention comprises a cosmetically acceptable base comprising at least 0.1% of a fragrance or a perfumery molecule.

The term "fragrance" is defined as odoriferous compounds or a mixture of odoriferous compounds, optionally mixed with a suitable solvent diluent or carrier, which is employed to impart a desired odor. A perfumery molecule or a group of perfumery molecules formulated as a perfume is generally incorporated in most personal care compositions. Any perfumery molecule / fragrance which is cosmetically acceptable may be included in the composition of the invention.

Fragrance components and mixtures thereof may be obtained from natural products such as essential oils, absolutes, resinoids, resins and concretes, as well as synthetic products such as hydrocarbons, alcohols, aldehydes, ketones, ethers, carboxylic acids, esters, acetals, ketals, nitriles and the like, including saturated and unsaturated compounds, aliphatic, carbocyclic and heterocyclic compounds.

Suitable characteristics of fragrances can include one or more of lavender, violet, rose, jasmin, pine, woody, floral, fruity, lemon, lime, apple, peach, raspberry, strawberry, banana, plum, apricot, vanilla, pear, eucalyptus, aromatic, aldehydic, tutti frutti, oriental, sweet, amber, Paola, Muguet and Citronella (lime).

Typical fragrance components which may be are one or more of: 2-methoxy naphthalene; allyl cyclohexane propionate; alpha-citronellal; alpha-ionone; alpha-santalol; alpha-terpineol; ambrettolide; amyl benzoate; amyl cinnamate; amyl cinnamic aldehyde; aurantiol; benzaldehyde; benzophenone; benzyl acetate; benzyl salicylate; beta-caryophyllene; beta-methyl naphthyl ketone; cadinene; cavacrol; cedrol; cedryl acetate; cedryl formate; cinnamyl cinnamate; cis-jasmone; coumarin; cyclamen aldehyde; cyclohexyl salicylate; d-limonene; delta-nonalactone; delta-undecalactone; dihydro isojasmonate; dihydro mycenol; dimethyl acetal; diphenyl methane; diphenyl oxide; dodecalactone; ethyl methyl phenyl glycidate; ethyl undecylenate; ethylene brassylate; eugenol; exaltolide; galaxolide; gamma-n-methyl ionone; gamma-undecalactone; geranial; geranyl acetate; geranyl anthranilate; geranyl phenyl acetate; hexadecanolide; hexenyl salicylate; hexyl cinnamic aldehyde; hexyl salicylate; hydroxycitronellal; indole; iso E super; iso-amyl salicylate; iso-bornyl acetate; iso-butyl quinoline; iso- eugenol; laevo-carvone; lilial (p-t-bucinal); linalool; linalyl acetate; linalyl benzoate; methyl cinnamate; methyl dihydrojasmonate; methyl-N-methyl anthranilate; musk indanone; musk ketone; musk tibetine; myristicin; nerol; oxahexadecanolide-10; oxahexadecanolide-11 ; para-cymene; para-tert-butyl cyclohexyl acetate;; patchouli alcohol; phantolide; phenyl ethyl alcohol; phenyl ethyl benzoate; phenyl heptanol; phenylhexanol; phexylethylphenylacetate; thibetolide; vanillin; vertenex; vetiveryl acetate; yara-yara; and ylangene.

Suitable solvents, diluents or carriers for perfumes as mentioned above are, for example, ethanol, isopropanol, diethylene glycol monoethyl ether, dipropyl glycol, triethyl citrate and the like.

Highly preferred fragrance components used in cosmetic compositions are cyclic and acyclic terpenes and terpenoids. These materials are based upon isoprene repeating units. Examples include alpha and beta pinene, myrcene, geranyl alcohol and acetate, camphene, dl-limonene, alpha and beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen- 1-ol, terpin-4-ol, dihydroterpineol, methyl chavicol, anethole, 1 ,4-and 1 ,8-cineole, geranyl nitrile, isobornyl acetate, linalyl acetate, caryophyllene, alpha cedrene, guaiol, patchouli alcohol, alpha and beta santalol and mixtures thereof.

As per the present invention, amounts of the fragrance may range from 0.1 to 5%, usually from 0.1 to 1.5%, more usually from 0.5 to 0.8% by weight of the composition. Other components commonly included in conventional antiperspirant compositions may also be incorporated in the compositions of the present invention. Such components include skin care agents such as emollients, humectants and skin barrier promoters; skin appearance modifiers such as skin lightening agents and skin smoothing agents; anti-microbial agents, in particular organic anti-microbial agents, and preservatives.

The composition of the invention can be applied cosmetically and topically to the skin, broadly speaking, by one of two methods. Different consumers prefer one method or the other. In one method, sometimes called a contact method, a composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

### Stick or soft solid compositions

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water-soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

### Aerosol compositions

The composition may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquefied by compression, ii) volatile ethers and iii) compressed non-oxidising gases.

Class i) is conveniently a low boiling point material, typically boiling below -5 °C, and often below -15 °C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax. When the composition comprises a propellant, it is delivered as an aerosol.

The composition of the present invention can comprise a wide range of other optional components. The CTFA Personal Care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, conditioners, exfoliating agents, pH adjusters, preservatives, natural extracts, essential oils, skin sensates, skin soothing agents, and skin healing agents.

In accordance with another aspect is disclosed a method of providing antiperspirant and/or anti-inflammation to a topical surface of a body comprising the step of applying a personal care composition of the first aspect.

In accordance with another aspect is disclosed use of a compound of the Formula 1 in the manufacture of a personal care composition for reduction of sweat.

In accordance with a further aspect is disclosed the use of a compound of Formula 1 as an antiperspirant active.

The skin surface could be any topical surface which is prone to sweating especially the axilla i.e. the underarm portion of the human body. The method is preferably non-therapeutic.

The invention will now be demonstrated with the help of the following non-limiting examples.

### Examples

### Examples 1-3: Compounds as per the invention (as per formula 2, 4 and 5) were tested for anti-perspirancy activity using the invitro model described below.

### Isolation of human eccrine sweat glands

Viable human eccrine sweat glands were isolated from samples of human skin (obtained under ethical consent).

### Measurement of intracellular Ca²⁺ concentration

Intracellular [Ca²⁺]ᵢ was measured in isolated eccrine glands by plating them onto Matrigel coated glass coverslips and loading them with the calcium-sensitive, fluorescent dye Fura-2 by incubation (30 - 45 minutes, 37 °C) with the membrane-permeant, acetoxymethyl ester form of the dye (Fura-2AM). These coverslips (with dye-loaded glands) were then mounted in a small chamber attached to the stage of an inverted microscope, and the glands superfused (ca. 5 ml minute⁻¹, 37 °C) with physiological salt solution (PSS; composition (mM): NaCl 130, KCl 5, MgCl₂ 1, CaCl₂ 1, HEPES 20, D-Glucose 10, pH 7.4 with NaOH). Changes in [Ca²⁺]ᵢ were detected using 340 / 380 nm excitation and 510 nm emission wavelengths, and data expressed as the 340:380 ratio.

### Measurement of store-operated Ca²⁺ entry (SOCE)

To measure store-operated calcium entry (SOCE), independent of external membrane receptor activation, the following assay protocol was used. First, isolated glands were exposed to 1 µM thapsigargin in Ca²⁺-free PSS for 3 minutes. As thapsigargin inhibits the Ca²⁺-ATPase uptake pathway in intracellular Ca²⁺ stores, this enables Ca²⁺ to leak out of the store and subsequently trigger the activation of SOCE. In Ca²⁺-free PSS, this process is represented by a very small rise in [Ca²⁺]ᵢ. Glands were then exposed to 1 µM thapsigargin in Ca²⁺-containing PSS for 2 minutes, thereby inducing a rapid increase in [Ca²⁺]ᵢ via Ca²⁺ entry into the cells via the open SOCE pathway. To complete the protocol, thapsigargin is finally removed by washing the glands with Ca²⁺-containing PSS, and allowing the resting [Ca²⁺]ᵢ level to re-establish itself, and the intracellular Ca²⁺ stores to refill. The effectiveness of selected SOCE channel inhibitors were assessed by repeating the above procedure in the presence of the inhibitor (at a chosen concentration) from the time at which thapsigargin was added to release the intracellular Ca²⁺ stores, until the cells were finally washed following the Ca²⁺ peak initiated by Ca²⁺ entry. Dose-response curves were constructed for each inhibitor and the half-maximal concentration (IC₅₀) determined. The potency of each inhibitor was also assessed by expressing the reduction in the 340:380 ratio observed at the maximum concentration (10⁻⁵M) as a % of the control response (i.e. in the absence of inhibitor; see Table 1).

Table 1: Effect of selected SOCE channel inhibitors on store-operated Ca²⁺ entry. The table shows the IC₅₀ and the potency (reduction in 340:380 ratio at maximal inhibitor concentration at 10⁻⁵ M expressed as a % of the control value) of each inhibitor.

**Table 1**

| Examples | Compound | IC₅₀ | Potency (% inhibition at 10⁻⁵ M) |
|---|---|---|---|
| 1 | Formula 4 | -7.04 | 75.4 |
| 2 | Formula 5 | -6.86 | 76.0 |
| 3 | Formula 2 | -7.62 | 99.0 |

The low IC₅₀ values for all compounds in Table 1 show that they are highly effective in reducing the magnitude of the intracellular Ca²⁺ signal responsible for driving fluid secretion in eccrine sweat gland secretory coil cells (i.e. each compound is efficacious at a low half-maximal concentration). The compound denoted Formula 2 was the most potent as it reduced the magnitude of the Ca²⁺ signal by 99% at a concentration of 10⁻⁵ M. This means that the Ca²⁺ signal was nearly completely abolished in isolated eccrine gland cells which is expected to significantly reduce sweat secretion.

### Example A, 4-6: Anti-inflammatory properties of the compounds synthesized (Compound of formula 4 and 5) by way of THP-1 invitro assay

The following procedure was used to test the anti-inflammation efficacy of the actives.

THP1-XBIue™ (Cat No: thpx-sp, InvivoGen) cells were cultured as a suspension in RPMI 1640 medium supplemented with 10% FBS, penicillin (10 U/mL) - streptomycin (10 µg/ML). Cells were differentiated in 24-well plates at the density of 5 × 10⁵ cells / well with 100 nM PMA for 72 hours. Cells were then co-treated with pure *E. coli* lipopolysaccharides (LPS) and with active. After 24 hours, the supernatants were collected and measured for interleukin (IL)-6 as pro-inflammatory bio-marker using enzyme-linked immunosorbent assay (ELISA).

The results in terms of concentration of IL-6 in pg/ml is given in Table -2 below:

**Table -2**

| Examples | Composition | Concentration of IL-6 (pg/ml) | Standard Deviation |
|---|---|---|---|
| A | LPS | 24176 | 1433 |
| 4 | 20 µM of compound of formula 4 | 18218 | 2920 |
| 5 | 10 µM of compound of formula 5 | 14880 | 1001 |
| 6 | 20 µM of compound of formula 5 | 9142 | 1290 |

The data in Table - 2 above indicates that the compounds as per the present invention also provide for anti-inflammation benefit.

### Synthesis of compound with Formula 2

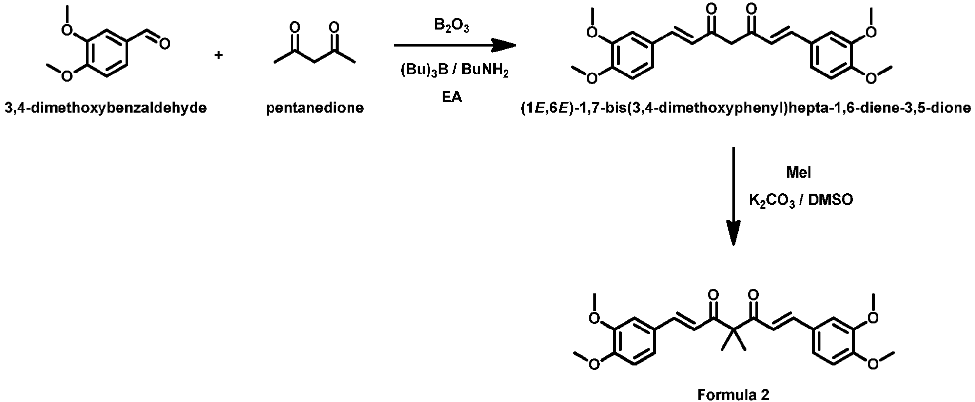

2,4-Pentanedione (3 g, 30.0 mmol) and boric anhydride (1.46g, 21.0mmol) were dissolved in ethyl acetate (EA) (30 ml) and heated at 45 °C for 30 minutes. 3,4-Dimethoxybenzaldehyde (9.96 g, 59.9mmol) and tributylborate (13.79 g, 59.9 mmol) were added and the mixture stirred at 45 °C for 30 minutes. Butylamine (4.44 ml, 44.9 mmol) in EA (30 ml) was added dropwise over 15 minutes and the solution stirred at 45 °C for 16 hours. Aqueous HCI (prepared by adding 4.5 ml conc. HCI to 25.5 ml water) was added and the biphasic mixture stirred and heated at 60 °C for 1 hour. The layers were separated and the aqueous layer extracted with EA (50 ml). The combined organic layers were washed with water, saturated NaCl, dried with Na₂SO₄, filtered and the solvents removed under reduced pressure to give crude product as a red oil (10.42 g). The crude product was purified by FC on silica gel eluting with EA:hexane (50:50) to give (1E,6E)-1,7-bis(3,4-dimethoxyphenyl)hepta-1,6-diene-3,5-dione as an orange solid (4.0 g, 34%). HPLC-UV showed >97% purity. ¹H NMR (60 MHz, CDCl₃), 16.05 (1H, bs), 7.59 (2H, d), 6.76-7.26 (6H, m), 6.45 (2H, d), 5.79 (1H, s), 3.91 (6H, s), 3.89 (6H, s).

Potassium carbonate (K₂CO₃) (1.74 g, 12.6mmol) was added to a solution of (1E,6E)-1,7-bis(3,4-dimethoxyphenyl) hepta-1,6-diene-3,5-dione (1g, 2.52 mmol) in anhydrous dimethylsulfoxide (DMSO) (5 ml), followed by iodomethane (Mel) (0.79ml, 12.6mmol) and the mixture stirred at room temperature (25 °C) for 24 hour. At this time, TLC [(30 µL aliquot into saturated NaCl:EA (400 µL:400 µL); eluting with EA:hexane (45:55)] showed the formation of a major product. The mixture was stirred for an additional 24 hours and partitioned between EA (30 ml) and saturated sodium chloride (NaCl) (30 ml). The organic layer was dried with Na₂SO₄, filtered and the solvents removed under reduced pressure to give an orange oil (1.2 g). The crude product was purified by FC on silica gel eluting with EA:hexane (45:55) to give pure product Formula 2 as a pale yellow solid (895 mg, 85%). HPLC-UV showed >97% purity; LC-MS (ESI+) showed expected mass [M+H]+ 425.4 (100%); ¹H NMR (60 MHz, CDCl₃), 7.64 (2H, d), 6.71-7.22 (6H, m), 6.58 (2H, d), 3.89 (6H, s), 3.84 (6H, s), 1.43 (6H, s).

### Synthesis of compound of Formula 4

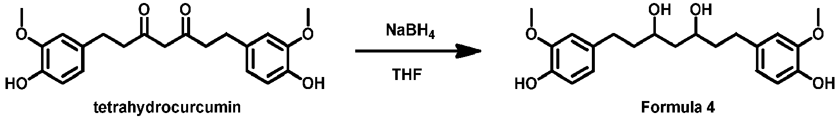

Sodium borohydride (230 mg, 6.0 mmol) was added to a solution of tetrahydrocurcumin (1.12 g, 3.0 mmol) in anhydrous tetrahydrofuran (THF) (15 mL) and the solution stirred at room temperature for 5 hours. At this time, TLC [(30 µL aliquot into saturated NH₄Cl:EA (400 µL:400 µL); eluting with EA:hexane (75:25)], showed the formation of a major product. The reaction mixture was diluted with 0.3 N HCI (150 ml) and extracted with EA (3 X 50 mL). The organic layer was dried with Na₂SO₄, filtered and the solvents removed under reduced pressure to give a colorless oil (1.2 g). The crude product was purified by FC on silica gel eluting with EA:hexane (80:20) to give pure product formula 4 as a colorless gel (1.0g, 89%). HPLC-UV showed >99% purity; LC-MS (ESI+) showed expected mass [M+H]⁺ 341.1 (-2H₂O); ¹H NMR (400 MHz, CDCl₃) □ 6.67-6.81 (6H, m), 3.99 (1H, m), 3.90 (1H, m), 3.87 (6H, s), 2.61-2.69 (4H, m), 1.61-1.78 (6H, m).

### Synthesis of compound of Formula 5

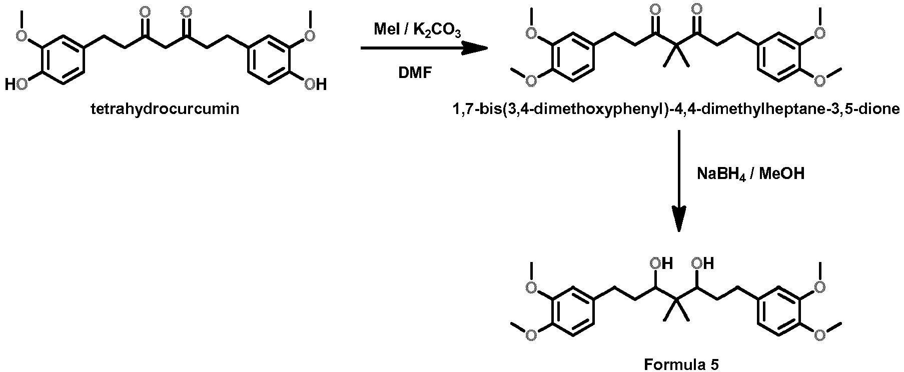

Potassium carbonate (11.1 g, 80.6 mmol) was added to a solution of tetrahydrocurcumin (6 g, 16.1 mmol) in anhydrous dimethylformamide (DMF) (45ml), followed by Mel (5.0 ml, 80.6 mmol) and the mixture stirred at room temperature for 72 hours. At this time, TLC [20 uL aliquot into 1N HCl: EA (400 uL:400 uL); eluting with EA:hex:DCM (5:30:65)] showed the formation of products and no starting material. The reaction mixture was partitioned between EA (400 ml) and water (400 ml), the layers separated, and the organic layer washed with saturated NaCl (200 ml), dried with Na₂SO₄, filtered and the solvents removed under reduced pressure to give a yellow oil (7.2 g). The crude product was purified by FC on silica gel eluting with EA:hex:DCM (5:30:65) to give pure product 1,7-bis(3,4-dimethoxyphenyl)-4,4-dimethylheptane-3,5-dione as a colorless oil which crystallized on standing (5.43g, 79%). HPLC-UV showed >99% purity; LC-MS (ESI+) showed expected mass [M+H]⁺ 429.5; ¹H NMR (60 MHz, CDCl₃), 6.62-6.67 (6H, m), 3.80 (6H, s), 3.77 (6H, s), 2.64 (4H, t), 2.61 (4H, t), 1.21 (6H, s).

Sodium borohydride (1.10 g, 29.2 mmol) was added to a solution of 1,7-bis(3,4-dimethoxyphenyl)-4,4-dimethylheptane-3,5-dione (2.5g, 5.8 mmol) in MeOH (60 mL) and the solution stirred at room temperature for 30 minutes. At this time, TLC [(20□L aliquot into saturated NH₄Cl:EA (400 µL:400 µL); eluting with EA:hexane (1:1)], showed the clean formation of product. The reaction mixture_was poured into ice cold saturated NH₄Cl (200ml), followed by addition of DCM (200 ml) and the mixture stirred vigorously for 5 minutes. The layers were separated, and the organic layer filtered through Na₂SO₄ and the solvents removed under reduced pressure to give a colorless gel (2.6 g). The crude product was purified by FC on silica gel eluting with EA:hexane (1:1) to give pure product Formula 5 as a colorless solid (2.45 g, 97%). HPLC-UV showed >97% purity; LC-MS (ESI+) showed expected mass [M+H]⁺ 433.5; ¹H NMR (60 MHz, CDCl₃), 6.75-6.89 (6H, m), 3.49-4.01 (15H, m), 2.61-2.87 (4H, m), 1.62-2.03 (4H, m), 095-0.97 (3H, m), 0.79-0.80 (3H, m).

## Claims

1. A personal care composition comprising:
(i) a compound of the Formula 1
Ar-CHₙCHₙ-X.C(R)₂-X.CHₙCHₙ-Ar (Formula 1)
wherein Ar is a substituted or unsubstituted phenyl group;
R is H or CH₃;
X is CH(OH) group or C=O group;
n has the value 1 or 2; and,
(ii) a topically acceptable base comprising at least 0.1% of a fragrance wherein,
when n=1, the compound of (Formula 1) is (1E,6E)-1,7-bis(3,4-dimethoxyphenyl)-4,4-dimethylhepta-1,6-diene-3,5-dione (Formula 2), 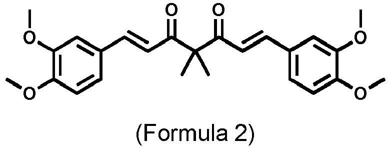 and when n=2, the compound of (Formula 1) is 1,7-bis(4-hydroxy-3-methoxyphenyl) heptane-3,5-diol (Formula 4) 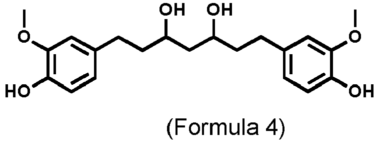 or is 1,7-bis (3,4-dimethoxyphenyl)-4,4-dimethylheptane-3,5-diol (Formula 5).

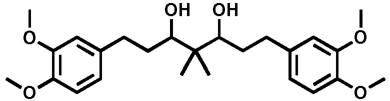

2. A composition as claimed in claim 1 wherein the compound of formula 2 is prepared by process comprising the steps of:
(i) (step 1): condensation of 3,4-dimethoxybenzaldehyde with 2,4-pentanedione to generate (1E,6E)-1,7-bis(3,4-dimethoxyphenyl)hepta-1,6-diene-3,5-dione; and,
(ii) (step 2): methylation of (1E,6E)-1,7-bis(3,4-dimethoxyphenyl)hepta-1,6-diene-3,5-dione to generate compound with Formula 2.

3. A composition as claimed in claim 2 wherein the compound of Formula 4 is prepared by a process comprising the step of reducing tetrahydrocurcumin using (i) a reducing agent or (ii) via hydrogenation.

4. A composition as claimed in claim 1 wherein compound of Formula 5 is prepared by a process comprising the steps of:
(i) (step 1): methylation of tetrahydrocurcumin with a methylating agent to generate 1,7-bis(3,4-dimethoxyphenyl)-4,4-dimethylheptane-3,5-dione; and,
(ii) (step 2): reduction of 1,7-bis(3,4-dimethoxyphenyl)-4,4-dimethylheptane-3,5-dione using a reducing agent to generate compound with Formula 5.

5. A composition as claimed in any of claims 1 to 4 wherein said composition is in the form of a roll-on, a propellant-containing composition, a gel or a stick.

6. A personal care composition as claimed in any of claims 1 to 5 for use in the therapeutic treatment of hyperhidrosis.

7. Cosmetic, non-therapeutic use of a compound selected from (1E,6E)-1,7-bis(3,4-dimethoxyphenyl)-4,4-dimethylhepta-1,6-diene-3,5-dione (Formula 2); 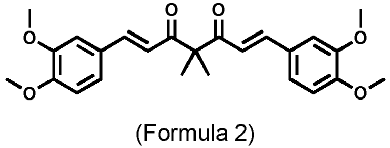 1,7-bis(4-hydroxy-3-methoxyphenyl) heptane-3,5-diol (Formula 4): 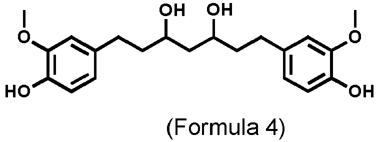 or 1,7-bis (3,4-dimethoxyphenyl)-4,4-dimethylheptane-3,5-diol (Formula 5) 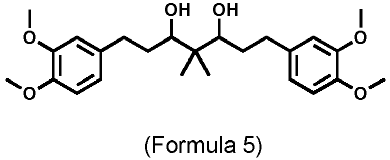 as an antiperspirant active.

## Patentansprüche

1. Körperpflegezusammensetzung, umfassend:
(i) eine Verbindung der Formel 1
Ar-CHₙCHₙ-X.C(R)₂-X.CHₙCHₙ-Ar (Formel 1)
worin Ar eine substituierte oder unsubstituierte Phenylgruppe ist;
R H oder CH₃ ist;
X die Gruppe CH(OH) oder die Gruppe C=O ist;
n den Wert 1 oder 2 aufweist; und
(ii) eine topisch akzeptable Basis, umfassend mindestens 0,1% eines Duftstoffs,
worin,
wenn n = 1, die Verbindung von (Formel 1) (1E,6E)-1,7-Bis (3,4-dimethoxyphenyl)-4,4-dimethylhepta-1,6-dien-3,5-dion (Formel 2) ist, 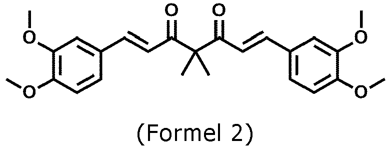 und wenn n = 2 ist, die Verbindung von (Formel 1) 1,7-Bis(4-hydroxy-3-methoxyphenyl)heptan-3,5-diol (Formel 4) ist, 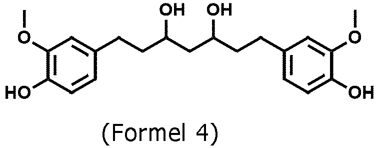 oder 1,7-Bis (3,4-dimethoxyphenyl)-4,4-dimethylheptan-3,5-diol (Formel 5) ist

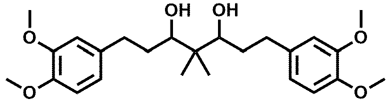

2. Zusammensetzung wie in Anspruch 1 beansprucht, wobei die Verbindung der Formel 2 durch ein Verfahren hergestellt wird, das die folgenden Schritte umfasst:
(i) (Schritt 1): Kondensation von 3,4-Dimethoxybenzaldehyd mit 2,4-Pentandion, um (1E, 6E)-1,7-Bis (3,4-dimethoxyphenyl)hepta-1,6-dien-3,5-dion zu bilden; und,
(ii) (Schritt 2): Methylierung von (1E,6E)-1,7-Bis(3,4-dimethoxyphenyl)hepta-1,6-dien-3,5-dion, um Verbindung mit der Formel 2 zu bilden.

3. Zusammensetzung wie in Anspruch 2 beansprucht, wobei die Verbindung der Formel 4 durch ein Verfahren hergestellt wird, das den Schritt des Reduzierens von Tetrahydrocurcumin unter Verwendung von (i) einem Reduktionsmittel oder (ii) über Hydrierung umfasst.

4. Zusammensetzung wie in Anspruch 1 beansprucht, wobei die Verbindung der Formel 5 durch ein Verfahren hergestellt wird, umfassend die Schritte:
(i) (Schritt 1): Methylierung von Tetrahydrocurcumin mit einem Methylierungsmittel, um 1,7-Bis(3,4-dimethoxyphenyl)-4,4-dimethylheptan-3,5-dion zu bilden; und
(ii) (Schritt 2): Reduktion von 1,7-Bis(3,4-dimethoxyphenyl)-4,4-dimethylheptan-3,5-dion unter Verwendung eines Reduktionsmittels, um Verbindung mit Formel 5 zu bilden.

5. Zusammensetzung wie in irgendeinem der Ansprüche 1 bis 4 beansprucht, wobei die Zusammensetzung in Form eines Deorollers, einer Treibmittel enthaltenden Zusammensetzung, eines Gels oder eines Stifts vorliegt.

6. Körperpflegezusammensetzung, wie in irgendeinem der Ansprüche 1 bis 5 beansprucht, zur Verwendung bei der therapeutischen Behandlung von Hyperhydrose.

7. Kosmetische, nichttherapeutische Verwendung einer Verbindung ausgewählt aus (1E,6E)-1,7-Bis(3,4-dimethoxyphenyl)-4,4-dimethylhepta-1,6-dien-3,5-dion (Formel 2) 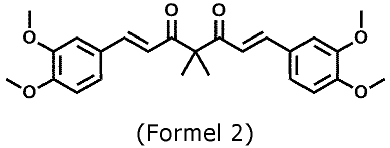 1,7-Bis(4-hydroxy-3-methoxyphenyl)heptan-3,5-diol (Formel 4) 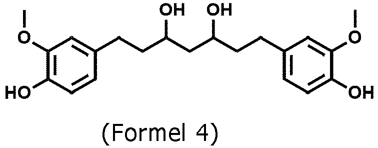 oder 1,7-Bis (3,4-dimethoxyphenyl)-4,4-dimethylheptan-3,5-diol (Formel 5) 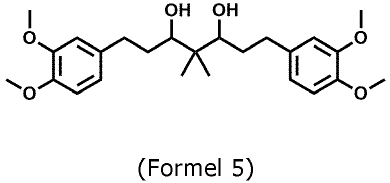 als ein Antiperspirant-Wirkstoff.

## Revendications

1. Composition pour le soin de la personne comprenant :
(i) un composé de la Formule 1
Ar-CHₙCHₙ-X.C(R)₂-X.CHₙCHₙ-Ar (Formule 1)
dans laquelle Ar est un groupe phényle substitué ou non substitué ;
R est H ou CH₃ ;
X est le groupe CH(OH) ou groupe C=O ;
n présente la valeur 1 ou 2 ; et,
(ii) une base topiquement acceptable comprenant au moins 0,1 % d'un parfum
dans laquelle,
lorsque n=1, le composé de (Formule 1) est la (1E,6E)-1,7-bis(3,4-diméthoxyphényl)-4,4-diméthylhepta-1,6-diène-3,5-dione (Formule 2), 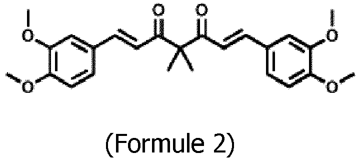 et lorsque n=2, le composé de (Formule 1) est le 1,7-bis(4-hydroxy-3-méthoxyphényl)heptane-3,5-diol (Formule 4) 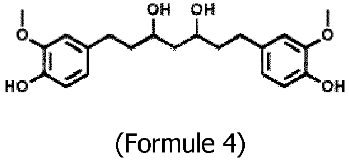 ou le 1,7-bis(3,4-diméthoxyphényl)-4,4-diméthylheptane-3,5-diol (Formule 5).

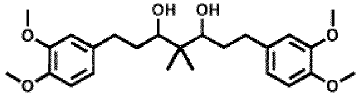

2. Composition selon la revendication 1, dans laquelle le composé de Formule 2 est préparé par un procédé comprenant les étapes de :
(i) (étape 1) : condensation de 3,4-diméthoxybenzaldéhyde avec de la 2,4-pentanedione pour produire la (1E,6E)-1,7-bis(3,4-diméthoxyphényl)hepta-1,6-diène-3,5-dione ; et,
(ii) (étape 2) : méthylation de (1E,6E)-1,7-bis(3,4-diméthoxyphényl)hepta-1,6-diène-3,5-dione pour produire le composé avec la Formule 2.

3. Composition selon la revendication 2, dans laquelle le composé de Formule 4 est préparé par un procédé comprenant l'étape de réduction de tétrahydrocurcumine en utilisant (i) un agent de réduction ou (ii) via hydrogénation.

4. Composition selon la revendication 1, dans laquelle le composé de Formule 5 est préparé par un procédé comprenant les étapes de :
(i) (étape 1) : méthylation de tétrahydrocurcumine avec un agent de méthylation pour produire la 1,7-bis(3,4-diméthoxyphényl)-4,4-diméthylheptane-3,5-dione ; et,
(ii) (étape 2) : réduction de 1,7-bis(3,4-diméthoxyphényl)-4,4-diméthylheptane-3,5-dione en utilisant un agent réducteur pour produire le composé avec la Formule 5.

5. Composition selon l'une quelconque des revendications 1 à 4, dans laquelle ladite composition se trouve dans la forme d'une bille, une composition contenant un agent propulseur, un gel ou un stick.

6. Composition pour le soin de la personne selon l'une quelconque des revendications 1 à 5 pour une utilisation dans le traitement thérapeutique de l'hyperhidrose.

7. Utilisation cosmétique, non-thérapeutique d'un composé choisi parmi la (1E,6E)-1,7-bis(3,4-diméthoxyphényl)-4,4-diméthylhepta-1,6-diène-3,5-dione (Formule 2) ; 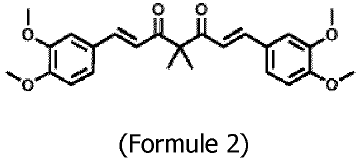 le 1,7-bis(4-hydroxy-3-méthoxyphényl)heptane-3,5-diol (Formule 4) : 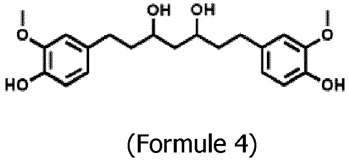 ou le 1,7-bis-(3,4-diméthoxyphényl)-4,4-diméthylheptane-3,5-diol (Formule 5) 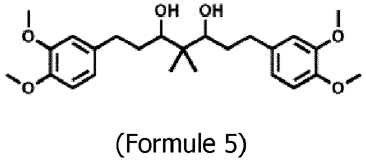 comme un actif antiperspirant.
